(19) Europäisches Patentamt
European Patent Office
Office européen des brevets

(11) Numéro de publication: **0 006 792**
**B1**

(12) **FASCICULE DE BREVET EUROPEEN**

(45) Date de publication du fascicule du brevet: **23.06.82**

(21) Numéro de dépôt: **79400382.2**

(22) Date de dépôt: **12.06.79**

(51) Int. Cl.³: **G 01 N 33/60**, A 61 K 43/00, C 07 B 23/00, C 07 C 103/52

(54) **Composé iodé, utilisable comme traceur en radioimmunologie, ainsi que son procédé de fabrication.**

(30) Priorité: **20.06.78 FR 7818404**

(43) Date de publication de la demande: **09.01.80 Bulletin 80/1**

(45) Mention de la délivrance du brevet: **23.06.82 Bulletin 82/25**

(84) Etats contractants désignés: **CH DE GB**

(56) Documents cités:
**FR - A - 1 527 279**
**PROSTAGLANDINS vol. 6, no. 1, 10 avril 1974, pages 137—148**
**LOS ALTOS, California, USA**
**S. OHKI et al.: "Radioimmunoassays of Prostaglandin F 2 alpha and Prostaglandin F 2 alpha-Main Urinary Metabolite with Prostaglandin-125 I-Tyrosine Methylester Amide"**

**INTERNATIONAL JOURNAL OF APPLIED RADIATION AND ISOTOPES, Vol. 26, 1975, pages 527—532**
**Pergamon Press (IE)**
**R. A. CARO et al.: "Labeling of Proteins with 125I and Experimental Determination of its Specific Activity".**

(73) Titulaire: **COMMISSARIAT A L'ENERGIE ATOMIQUE Etablissement de Caractère Scientifique Technique et Industriel**
**B.P. 510**
**F-75752 Paris Cedex 15 (FR)**

(72) Inventeur: **Delaage, Michel**
**22, Vallon de la Baudille**
**F-13007 Marseille (FR)**

(74) Mandataire: **Mongrédien, André et al,**
**c/o Brevatome 25, rue de Ponthieu**
**F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**EP 0 006 792 B1**

Composé iodé utilisable comme traceur en radioimmunologie, ainsi que són procédé de fabrication.

La présente invention, due aux travaux de Monsieur Michel DELAAGE du Centre National de la Recherche Scientifique, et du Commissariat à l'Energie Atomique, a pour objet un composé iodé utilisable comme traceur en radio-immunologie et le procédé de préparation dudit composé iodé. Le composé iodé conforme à l'invention est utilisable comme traceur en radio-immunologie, en tant qu'antigène marqué.

On rappelle que la plupart des antigènes donnent naissance à des anticorps spécifiques de l'antigène et se combinent à ces anticorps pour donner un complexe; il en va de même pour certains de leurs analogues dans lesquels est présentée l'intégrité des sites actifs antigéniques. La réaction antigène-anticorps est utilisée pour déterminer la quantité d'antigène dans un milieu donné; cette mesure quantitative, tout d'abord de la réaction antigène-anticorps, pour en déduire la quantité d'antigène dans un milieu donné, peut être effectuée notamment par la méthode de radio-immunologie selon laquelle on utilise un traceur. De façon plus précise, la méthode radio-immunologique consiste à étudier la réaction antigène-anticorps en présence d'un traceur obtenu en marquant l'antigène ou l'anticorps ou un de leurs analogues par une substance radioactive telle que par exemple, de l'iode 125. Parmi les modes opératoires possibles, on peut citer notamment la méthode dite de compétition avec antigène en excès par rapport à l'anticorps; dans ce cas l'antigène marqué et l'antigène non marqué entrent alors en compétition pour se lier à l'anticorps, en quantité limitée par rapport à la quantité d'antigène totale. L'antigène marqué et l'anticorps sont ajoutés en quantité connue dans différents tubes de dosage; des quantités connues croissantes d'antigène non marqué sont ajoutées dans le tube, ce qui permet de tracer une courbe d'étalonnage: en effet, à tout moment, l'antigène libre peut être séparé de l'antigène lié à l'anticorps et la répartition de la radioactivité entre l'antigène libre et le complexe lié est mesurée pour chaque tube.

On voit donc que, lors de l'utilisation de la méthode de radio-immunologie, la nature et les propriétés de l'antigène marqué sont d'une grande importance. Cet antigène marqué, de préférence à l'iode radioactif, est préparé soit par synthèse de l'antigène, puis iodation de l'antigène ainsi préparé, soit par iodation de l'antigène extrait d'un organe humain ou animal, soit par préparation d'un composé iodé analogue de l'antigène existant naturellement dans le milieu à analyser.

Le marquage à l'iode de l'antigène n'est pas toujours possible directement; il peut être nécessaire de modifier préalablement cet antigène pour l'introduction de l'iode.

De nombreuses préparations de différents antigènes marqués faisant appel à une modification avant introduction de l'iode ont déjà été étudiées et mises au point. On peut citer, notamment, comme antigènes marqués ceux obtenus par accrochage du tyrosine-méthyl-ester sur un antigène et marquage à l'iode radioactif; ce composé présente l'inconvénient d'être peu soluble dans l'eau. On peut citer également ceux obtenus par accrochage d'un réactif connu sous le nom de "Bolton-Hunter" constitué par un ester succinimide de l'acide hydroxyphényl-propionique, de formule:

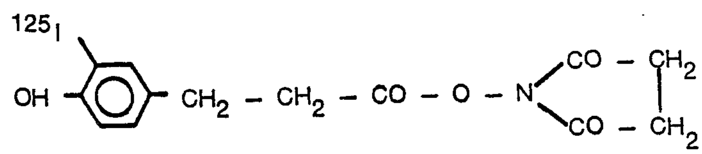

et qui est capable de se fixer à un antigène; ce composé a également l'inconvénient de ne pas être très soluble dans l'eau et, présente de plus, une affinité pour l'albumine, ce qui peut créer des anomalies lors de l'utilisation de la méthode de radio-immunologie.

L'invention a justement pour objet un composé iodé utilisable comme traceur en radio-immunologie qui pallie les inconvénients des composés rappelés ci-dessus; plus particulièrement le composé de l'invention présente l'avantage d'être très soluble dans l'eau et de permettre une bonne "reconnaissance" par l'anticorps lors de l'analyse par radio-immunologie, c'est-à-dire qu'il est tel que la réaction antigène marqué-anticorps soit le plus proche possible de la réaction antigène non marqué-anticorps.

Le composé iodé utilisable comme traceur en radioimmunologie en tant qu'antigène marqué, se caractérise en ce qu'il est constitué par un composé conjugué d'un dipeptide et d'un composé choisi parmi un antigène présentant une fonction carboxylique libre et l'acide 5-hydroxy-indole-acétique, ledit dipeptide étant constitué par un produit de condensation entre la tyrosine et un peptide, le groupement tyrosine dudit dipeptide étant marqué à l'iode radioactif.

Selon l'invention, le dipeptide est constitué par un produit de condensation de la tyrosine avec un peptide choisi dans le groupe comprenant la glycine, la leucine, l'isoleucine, l'alanine, la sérine, la tyrosine, la phénylalanine, la thréonine, la valine, l'histidine, la proline; ce peut être également un produit de condensation de la tyrosine avec la lysine, l'arginine, l'acide aspartique, l'acide glutamique, l'aspartamine, la glutamine.

De préférence, on prend la glycine comme peptide devant être associé à la tyrosine dans le dipeptide, ce qui donne le glycyl-tyrosine de formule:

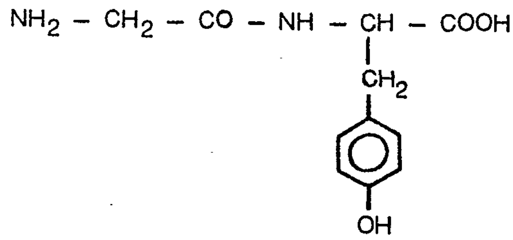

L'antigène conjugué avec le dipeptide pour former le composé traceur de l'invention peut être une hormone lipophile telle que l'hormone juvénile, une prostaglandine telle que la $PGE_2$, un neurotransmetteur tel que la sérotonine.

De même l'antigène conjugué avec le dipeptide pour former le composé traceur de l'invention peut être constitué par de l'acide abcissique (hormone végétale) ou par une progestérone.

Ainsi, avec le glycyl-tyrosine accroché sur l'hormone juvénile, le groupement tyrosine étant iodé, on a un composé de formule:

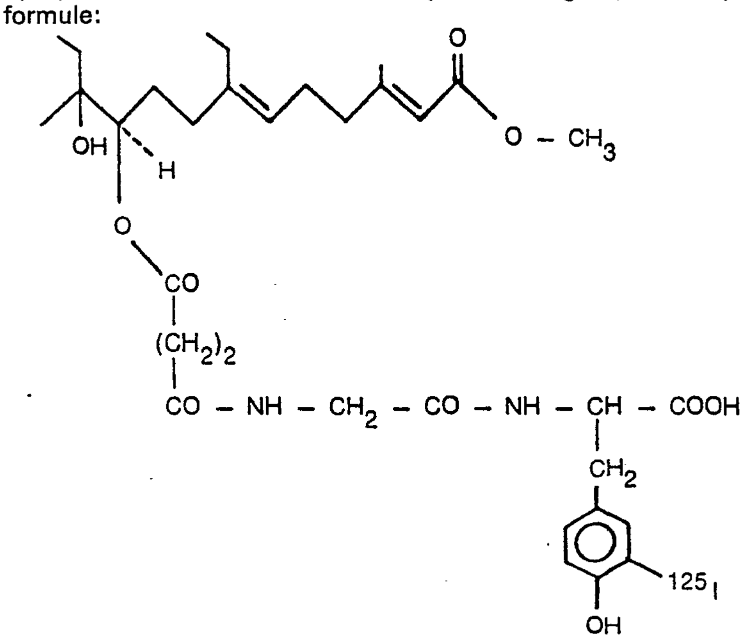

Avec le glycyl-tyrosine accroché sur la prostaglandine $PGE_2$, le groupement tyrosine étant iodé, on a un composé de formule:

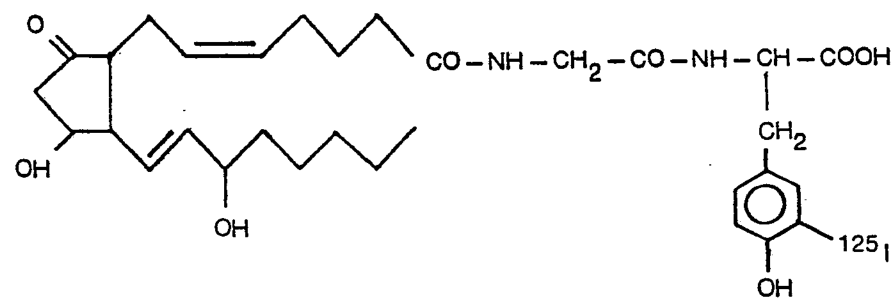

Pour obtenir un dérivé iodé analogue de la sérotonine, on accroche le glycyl-tyrosine sur l'acide 5-hydroxy-indole acétique, le groupement tyrosine étant iodé, et l'on obtient un composé traceur de formule:

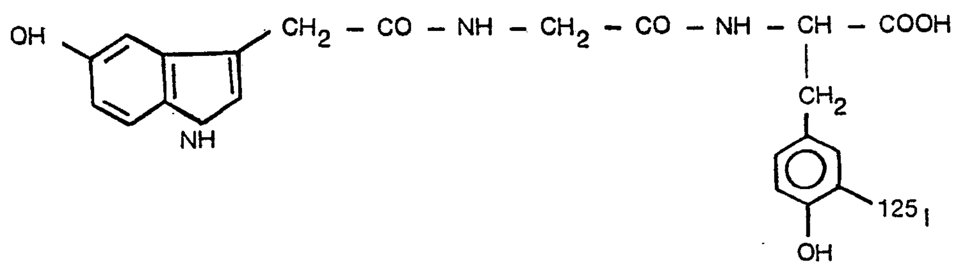

L'invention a également pour objet un procédé de préparation d'un composé iodé tel que défini ci-dessus. Ce procédé se caractérise en ce qu'il comporte une réaction de condensation d'un antigène ou de l'acide 5-hydroxy-indole-acétique avec un dipeptide, ledit dipeptide étant constitué par un produit de condensation entre la tyrosine et un peptide, et une réaction d'iodation du groupement tyrosine.

Selon un mode préféré de l'invention, l'antigène ou l'acide 5-hydroxy-indole-acétique est préalablement activé par du chloroformate d'éthyle.

Selon l'invention, on peut, soit effectuer, dans un premier temps, la réaction de condensation entre l'antigène et le dipeptide et, dans un deuxième temps, effectuer la réaction d'iodation du groupement tyrosine, soit effecteur, dans une premier temps, l'iodation du groupement tyrosine du dipeptide puis, dans un deuxième temps, la condensation du dipeptide ainsi marqué avec l'antigène ou l'acide 5-hydroxy-indole-acétique. Ce dernier mode de réalisation du procédé n'est employé que dans le cas où l'antigène seul ne supporte pas l'iodation.

Selon l'invention, la réaction d'iodation est une réaction de type classique selon laquelle on utilise l'iode 125 sous forme d'iodure de sodium et la chloramine T, et on arrête la réaction d'oxydo-réduction amenant la formation d'iode 125 et la fixation de cet iode 125 sur le groupement tyrosine du dipeptide à l'aide de métabisulfite de sodium. Les composés iodés obtenus sont purifiés ensuite par chromatographie sur le gel vendu sous la marque SEPHADEX G 25.

Le dipeptide tyrosine-peptide est préparé préalablement par toute méthode connue permettant la liaison entre deux peptides, par exemple par usage du carbodiimide.

L'invention sera mieux comprise à la lecture de la description qui suit, donnant des exemples de préparation de composés iodés et se référant au dessin annexé sur lequel:

— la Fig. 1 illustre le chromatogramme obtenu lors de la purification sur colonne de SEPHADEX G 25 du composé obtenu dans l'exemple 3, c'est-à-dire d'un composé constitué par du glycyl L-tyrosine conjugué à de la succinyl sérotonine.

— la Fig. 2 illustre le chromatogramme obtenu par purification sur SEPHADEX G 25 du composé obtenu dans l'exemple 4, c'est-à-dire d'un composé iodé constitué par de la sérotonine marquée à l'iode réactif par réaction avec le réactif de Bolton-Hunter.

— la Fig. 3 illustre le chromatogramme obtenu lors de la purification sur SEPHADEX G 25 du composé iodé obtenu dans l'exemple 5, c'est-à-dire d'un composé constitué par du glycyl L-tyrosine conjugué à un dérivé succinylé de l'hormone juvénile, et

— la Fig. 4 illustre le chromatogramme obtenu lors de la purification sur SEPHADEX G 25 du composé obtenu dans l'exemple 6, c'est-à-dire un composé iodé obtenu par réaction du dérivé succinylé de l'hormone juvénile avec du tyrosine-méthyl ester.

Parmi ces exemples, seuls les exemples 1, 2, 3 et 5 se rapportent à la préparation de composés iodés conformes à l'invention, les exemples 4 et 6 sont donnés uniquement à titre comparatif.

Exemple 1

On cherche à préparer un composé iodé constitué par le dipeptide glycyl-tyrosine iodé accroché à la prostaglandine $PGE_2$. On opère à 4°C.

On dissout 2 mg de prostaglandine $PGE_2$ dans 200 $\mu$l de diméthylformamide avec 20 $\mu$l d'une solution formée à partir de 100 $\mu$l de triéthylamine et 1 ml de diméthylformamide.

Au temps zéro, on a ajoute à cette solution 10 $\mu$l d'une solution formée à partir de 50 $\mu$l de éthylchloroformate et 750 $\mu$l de diméthylformamide.

Au temps 10 minutes, on ajoute 100 $\mu$l d'une solution obtenue à partir de 16 mg de dipeptide glycyltyrosine, 500 $\mu$l de diméthylformamide et 100$\mu$l d'une solution formée à partir de 100 $\mu$l de triéthylamine et 1 ml d'eau.

Au temps 15 minutes, on a joute 500 $\mu$l d'une solution tampon neutre de citrate 0,1 M de pH 6,2, pour arrêter la réaction.

On introduit la solution finale obtenue dans une colonne de chromatographie contenant du gel Sephadex, équilibrée dans une solution NaCl de concentration $2 \times 10^{-2}$ M.

On élue avec un gradient de 2 × 800 ml de NaCl $2 \times 10^{-2}$ M et NaCl 0,4 M.

On récupère, à la sortie de la colonne, le composé conjugué prostaglandine $PGE_2$-glycyl-tyrosine qui sort un peu avant le milieu du gradient d'élution. On rassemble les différentes fractions de $PGE_2$-glycyl-tyrosine, on mesure la concentration d'après la densité optique à 280 nm et on l'ajuste à $1,5 \times 10^{-4}$ M environ.

On effectue ensuite l'iodation du composé conjugué $PGE_2$-glycyl-tyrosine (1 $\mu$g) en ajoutant une solution de chloramine T (10 $\mu$g) et 1 à 2 millicuries de iodure de sodium, puis au bout d'un certain temps, du métabisulfite de sodium.

Exemple 2

On cherche à préparer un composé iodé, analogue de la sérotonine, constitué par du glycyl-tyrosine conjugué avec de l'acide 5-hydroxy-indole acétique, marqué à l'iode 125. On opère à 4°C.

On dissout 2 mg d'acide 5-hydroxy-indole acétique avec 200 $\mu$l de diméthylformamide et 20 $\mu$l d'une solution preparée à partir de 100 $\mu$l de triéthylamine et 1 ml de diméthylformamide.

Au temps zéro, on ajoute 10 $\mu$l d'une solution préparée à partir de 50 $\mu$l d'éthylchloroformate et

750 ml de diméthylformamide; on obtient ainsi une solution A.

Au temps 10 minutes, on effectue, séparément dans un autre tube, la iodation du dipeptide glycyl-tyrosine: on prépare une solution par mélange de 2 mg de glycyl-tyrosine et 2 ml d'eau; et on ajoute à 25 $\mu$l de cette solution, 1 ml de tampon phosphate 0,1 M de pH 7,5. A 30 $\mu$l de la solution ainsi obtenue, on ajoute 5 $\mu$l d'une solution de métabisulfite de sodium de concentration 0,6 mg/ml dans un tampon phosphate 0,1 M de pH 7,5, 20$\mu$l d'une solution de $^{125}$INa à 100 mCi/ml, puis 20 fois 1 $\mu$l d'une solution de chloramine T à 1 mg/ml pour une durée totale de 1 minute; ensuite, on ajoute 50 $\mu$l d'une solution de métasulfite de sodium à 0,6 mg/ml dans un tampon phosphate 0,1 M de pH voisin de 7,5 et 10 $\mu$l d'une solution préparée à partir de 100 $\mu$l de triéthylamine et 1 ml de diméthylformamide; on obtient ainsi une solution B.

Au temps 15 minutes, on ajoute 10 $\mu$l d'une solution préparée à partir de 10 $\mu$l de butylamine et 1 ml de diméthylformamide dans la solution A qui constitue le milieu réactionnel d'activation de l'acide 5-hydroxy-indole acétique.

Au temps 16 minutes, on ajoute les 240 $\mu$l de la solution A, additionnée de butylamine et diméthylformamide comme précédemment, aux 135 $\mu$l de la solution B qui constitue le mélange de iodation.

Au temps 21 minutes, on ajoute à la solution obtenue 500 $\mu$l de tampon citrate 0,1 M de pH voisin de 6,2.

On introduit la solution finale obtenue dans une colonne de chromatographie de gel SEPHADEX G 25, équilibrée dans le tampon citrate 0,1 M de pH voisin de 6,2. Le composé iodé conjugué d'acide 5-hydroxy-indole acétique et de glycyl-tyrosine est élué avec un volume trois fois plus grand que celui nécessaire à l'élution de l'iodure.

Exemple 3

Cet exemple se rapporte à la préparation d'un composé iodé constitué par du glycyl-tyrosine conjugué à de la succinyl-sérotonine.

Tout d'abord, on prépare le dérivé succinylé de la sérontonine de la façon suivante.

On mélange 40 mg de sérotonine, 90 mg d'anhydride succinique, 2 ml d'eau et 163 $\mu$l de KOH 9M. On agite le mélange pendant environ 5 mn pour obtenir la réaction, puis on le purifie sur une colonne SEPHADEX G 25 (2,5 × 80 cm) en réalisant l'élution au moyen d'eau distilliée. On recueille la fraction contenant le composé succinylé et on le lyophilise.

Ensuite, on prépare le composé iodé de la façon suivante, en opérant à 4°C.

On dissout 3 mg du dérivé succinylé de la sérotonine avec 200 $\mu$l de diméthyl-formamide et 20 $\mu$l d'une solution préparée à partir de 100 $\mu$l de triéthylamine et 1 ml de diméthylformamide.

Au temps zéro, on ajoute 10 $\mu$l d'une solution préparée à partir de 50 $\mu$l d'éthylchloroformate et 750 ml de diméthylformamide; on obtient ainsi une solution A.

Au temps 10 minutes, on effectue, séparément dans un autre tube, la iodation du dipeptide glycyl-tyrosine on prépare une solution par mélange de 2 mg de glycyl-tyrosine et 2 ml d'eau; et on ajoute à 25 $\mu$l de cette solution, 1 ml de tampon phosphate 0,1 M de pH 7,5. A 30 $\mu$l de la solution ainsi obtenue, on ajoute 5 $\mu$l d'une solution de métabisulfite de sodium de concentration 0,6 mg/ml dans un tampon phosphate 0,1 M de pH 7,5, 20 $\mu$l d'une solution de $^{125}$INa à 100 mCi/ml, puis 20 fois 1 $\mu$l d'une solution de chloramine T à 1 mg/ml pour une durée totale de 1 minute; ensuite, on ajoute 50 $\mu$l d'une solution de métabisulfite de sodium à 0,6 mg/ml dans un tampon phosphate 0,1 M de pH voisin de 7,5 et 10 $\mu$l d'une solution préparée à partir de 100 $\mu$l de triéthylamine et 1 ml de diméthylformamide; on obtient ainsi une solution B.

Au temps 15 minutes, on ajoute 10 $\mu$l d'une solution préparée à partir de 10 $\mu$l de butylamine et 1 ml de diméthylformamide dans la solution A qui constitue le milieu réactionnel d'activation du dérivé succinylé de la sérotonine.

Au temps 16 minutes, on ajoute les 240 $\mu$l de la solution A, additionnée de butylamine et diméthyl-formamide comme précédemment, aux 135 $\mu$l de la solution B, qui constitue le mélange de iodation.

Au temps 21 minutes, on ajoute à la solution obtenue 500 $\mu$l de tampon citrate 0,1 M de pH voisin de 6,2.

On introduit la solution finale obtenue dans une colonne de chromatographie de gel SEPHADEX G 25 (0,9 × 58 cm) équilibré dans le tampon citrate 0,1 M de pH voisin de 6,2 et on réalise l'élution au moyen du même tampon citrate en recueillant la solution éluée par fractions de 2,85 ml.

Les résultats obtenus sont donnés sur la Fig. 1 qui est un chromatogramme représentant l'évolution de la radioactivité dans les fractions recueillies successivement.

Sur cette figure, on voit que le composé iodé qui correspond au 3ème. pic est élué très rapidement puis-qu'il apparaît vers la 60ème. fraction.

Par ailleurs, on teste le composé iodé ainsi obtenu contre un immunsérum dilué entre $10^3$ et $10^4$, et l'on obtient un taux de fixation spécifique, ce composé pouvant être déplacé par de la sérotonine non marquée.

Exemple 4

Cet exemple se rapporte à la préparation d'un composé iodé obtenu par réaction de la sérotonine avec le réactif de Bolton-Hunter.

On rappelle que ce réactif qui est commercialisé par New England Nuclear sous la référence NEX 120, est constitué par une solution dans du benzène de l'ester succinimide de l'acide p-hydroxyphényl-propionique, cet ester étant marqué par de l'iode radioactif 125.

Pour obtenir le composé iodé de sérotonine, on fait réagir sous agitation 25 $\mu$l du réactif de Bolton-Hunter avec 10 $\mu$l d'un tampon borate contenant 2 $\mu$g de sérotonine.

On purifie le produit obtenu par chromatographie sur une colonne de SEPHADEX G 25 (0.9 x 57,5 cm) équilibré par un tampon citrate 0,1 M de pH 6,2 en réalisant l'élution par ce même tampon citrate et en recueillant la solution éluée par fractions de 2,9 ml au début de l'élution puis par fractions de 5,2 ml à partir de la 30ème fraction recueillie. La durée totale de l'élution étant de 70 heures.

Les résultats obtenus sont donnés sur la Fig. 2 qui représente l'évolution de la radioactivité dans les fractions recueillies successivement.

Au vu de cette figure, on voit que le chromatogramme présente plusieurs pics et que le plus important de ces pics qui correspond au produit de couplage de la sérotonine et du réactif de Bolton-Hunter, apparaît à partir de la 160ème fraction recueillie.

Ainsi, on constate que ce composé iodé sort très tard, après 3 jours environ d'élution.

Par ailleurs, en testant ce composé iodé contre un immunsérum dilué 60 fois en dialyse à l'équilibre, on obtient un taux de fixation de l'ordre de 50%.

Cependant, cette fixation n'est pas déplaçable par la sérotonine non marquée. Aussi, il semble que cette fixation soit due pour l'essentiel à l'albumine et non aux anticorps.

On constate ainsi que le réactif de Bolton-Hunter confère aux petites molécules une affinité pour l'albumine, ce qui constitue un obstacle majeur pour une utilisation du composé iodé obtenu à partir de ce réactif en radioimmunologie.

Exemple 5

Cet exemple se rapporte à la préparation d'un composé iodé constitué par du glycyl-L-tyrosine conjugué au dérivé succinylé de l'hormone juvénile.

Dans une première étape, on prépare le dérivé succinylé de l'hormone juvénile de la façon suivante.

On dissout 10 $\mu$l d'hormone juvénile qui se présente sous la forme d'un liquide huileux dans 1 ml de dioxane, puis on ajoute 40 $\mu$l d'acide sulfurique 0,5 N et on laisse reposer le mélange pendant une nuit à 37°C.

Ensuite, on neutralise par de la soude et on lyophilise le produit obtenu.

On dissout le produit lyophilisé dans 3 ml de dioxane et on ajoute à la solution 100 mg d'anhydride succinique et 500 $\mu$l de triéthylamine, puis on laisse reposer le mélange pendant 24 h à 37°C. On séche le milieu puis on le lyophilise et on reprend le produit lyophilisé dans 5 ml d'eau. On ajuste le pH à 6 et on purifie sur une colonne de chromatographie d'échange ions SEPHADEX QAE-A 25.

Le composé est élué par une solution de chlorure de sodium dont la concentration augmente au fur et à mesure de l'élution.

La solution contenant le dérivé succinylé de l'hormone juvénile est ensuite dessalée par passage dans une colonne de chromatographie identique à celle utilisée précédemment, l'élution étant réalisée dans ce cas au moyen d'acide chlorhydrique $10^{-2}$ M. On neutralise ensuite la solution éluée, puis on la lyophilise.

Dans une seconde étape, on prépare le composé iodé constitué par le glycyl L-tyrosine conjugué au dérivé succinylé de l'hormone juvénile ainsi obtenu de la façon suivante en opérant à 4°C.

On dissout 2 mg du dérivé succinylé de l'hormone juvénile obtenu précédemment dans 200 $\mu$l de diméthylformamide avec 20 $\mu$l d'une solution formée à partir de 100 $\mu$l de triéthylamine et 1 ml de diméthylformamide.

Au temps zéro, on ajoute à cette solution 10 $\mu$l d'une solution formée à partir de 50 $\mu$l d'éthyl-chloroformate et 750 $\mu$l de diméthylformamide.

Au temps 10 minutes, on ajoute 100 $\mu$l d'une solution obtenue à partir de 16 mg de dipeptide glycyl-tyrosine, 500 $\mu$l de diméthylformamide et 100$\mu$l d'une solution formée à partir de 100 $\mu$l de triéthylamine et 1 ml d'eau.

Au temps 15 minutes, on ajoute 500 $\mu$l d'une solution tampon neutre de citrate 0,1 M de pH 6,2 pour arrêter la réaction.

On introduit la solution finale obtenue dans une colonne de chromatographie contenant de gel SEPHADEX, équilibrée dans une solution NaCl de concentration $2 \times 10^{-2}$ M.

On élue avec un gradient de 2 x 800 ml de NaCl $2 \times 10^{-2}$ M et NaCl 0,4 M.

On récupère, à la sortie de la colonne, le composé conjugué succinyl hormone juvénile glycyl-tyrosine qui sort un peu avant le milieu du gradient d'élution. On rassemble les différentes fractions de succinylhormone juvénile glycyl-tyrosine. On mesure la concentration d'après la densité optique à

280 nm et on l'ajuste à 1,5 x $10^{-4}$ M environ.

On effectue ensuite l'iodation du composé conjugué dérivé succinylé de l'hormone juvénile — glycyl-L-tyrosine (1 $\mu$g) en ajoutant une solution de chloramine T (10 $\mu$g) et 1 à 2 millicuries d'iodure de sodium, puis au bout d'un certain temps, du métabisulfite de sodium.

Le composé iodé obtenu est ensuite purifié par chromatographie sur une colonne de SEPHADEX G 25 (1,5 x 95 cm) Pour cette chromatographie, on réalise l'élution au moyen d'un tampon citrate 0.1 M ayant un pH d'environ 6,2, et on recueille la solution éluée par fractions de 3,85 ml.

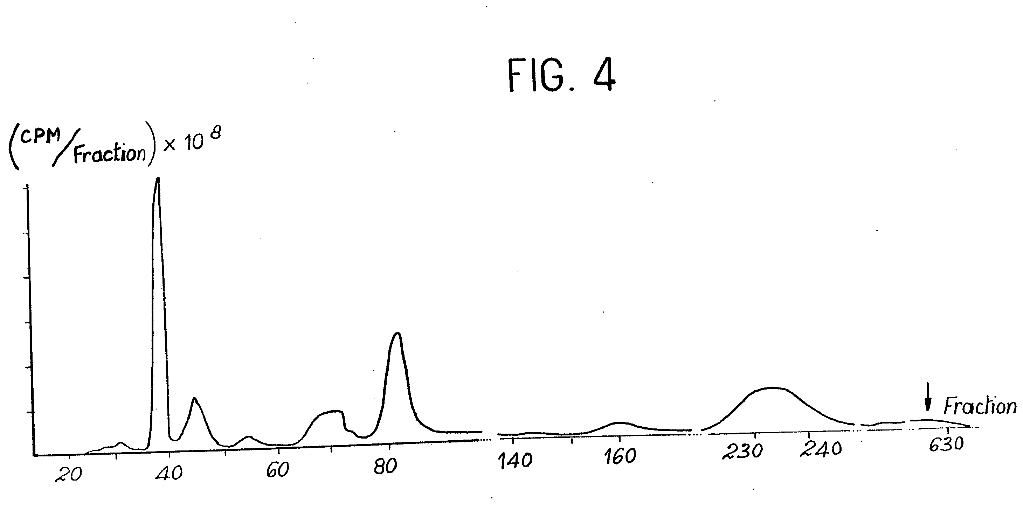

Les résultats obtenus sont donnés sur la Fig. 3 qui est un chromatogramme représentant l'évolution de la radioactivité dans les fractions recueillies.

Au vu de cette figure, on constate que le composé iodé obtenu par couplage du dérivé succinylé de l'hormone juvénile avec du glycyl-L-tyrosine est élué très rapidement.

Par ailleurs, il est très immunoréactif. En effet, on obtient 50% de fixation avec des immunsérums dont le taux de dilution est supérieur à $10^6$.

Enfin, on note que ce composé iodé se manipule sans problème dans des tubes et pipettes en plastique.

Exemple 6

Cet exemple se rapporte à la préparation d'un composé iodé obtenu par couplage tyrosine-méthyl-ester avec le dérivé succinylé de l'hormone juvénile obtenue lors de la première étape de l'exemple 5. Pour cette préparation, on opère à 4°C.

On dissout 2 mg du dérivé succinylé de l'hormone juvénile dans 200 $\mu$l de diméthylformamyde avec 20 $\mu$l d'une solution formée à partir de 100 $\mu$l de triéthylamine et 1 ml de diméthylformamide.

Au temps zéro, on ajoute à cette solution 10 $\mu$l d'une solution formée à partir de 50 $\mu$l d'éthyl-chloroformate et 750 $\mu$l de diméthylformamide.

Au temps 10 minutes, on ajoute 100 $\mu$l d'une solution obtenue à partir de 16 mg de tyrosine-méthyl ester, 500 $\mu$l de diméthylformamide et 100 $\mu$l d'une solution formée à partir de 100 $\mu$l de triéthylamine et 1 ml d'eau.

Au temps 15 minutes, on ajoute 500 $\mu$l d'une solution tampon neutre de citrate 0,1 M de pH 6,2 pour arrêter la réaction.

On introduit la solution finale obtenue dans une colonne de chromatographie contenant du gel SEPHADEX, équilibrée dans une solution NaCl de concentration 2x $10^{-2}$ M.

On élue avec un gradient de 2 x 800 ml de NaCl 2 x $10^{-2}$ M et NaCl 0,4 M.

On récupère, à la sortie de la colonne, le composé conjugué dérivé succinylé de l'hormone juvénile-tyrosine-méthyl ester qui sort un peu avant le milieu du gradient d'élution. On rassemble les différentes fractions de dérivé succinylé de l'hormone juvénile-tyrosine-méthyl ester, on mesure la concentration d'après la densité optique à 280 nm et on l'ajuste à 1,5 x $10^{-4}$ M environ.

On effectue ensuite l'iodation du composé conjugué dérivé succinylé de l'hormone juvénile-tyrosine-méthyl ester (1 $\mu$g) en ajoutant une solution de chloramine T (10 $\mu$g) et 1 à 2 millicuries d'iodure de sodium, puis au bout d'un cetain temps, du métabisulfite de sodium.

Après iodation, on purifie le composé iodé obtenu par chromatographie sur une colonne de SEPHADEX G 25 (1,5 x 90 cm) en réalisant l'élution au moyen d'un tampon citrate 0,1 M ayant un pH d'environ 6,2 et en recueillant la solution éluée par fractions de 4,5 ml.

Les résultats obtenus sont donnés sur la Fig. 4 qui est un chromatogramme représentant l'évolution de la radioactivité dans les fractions éluées successivement.

Au vu de cette figure, on constate que le composé iodé obtenu par couplage du tyrosine méthyl ester avec le dérivé succinylé de l'hormone juvénile sort difficilement de la colonne de SEPHADEX. En effet, la fraction immunoréactive qui correspond à ce composé apparaît seulement après une semaine d'élution, dans les fractions n° 559 à 625.

Contrairement au composé iodé obtenu dans l'exemple 4, ce dérivé iodé n'a pas d'affinité pour l'albumine. Cependant, ce composé iodé est très hydrophobe et s'accroche à tous les matériaux, surtout les plastiques. Aussi, son usage sur un plan quantitatif est pratiquement exclu.

Enfin, ce composé permet d'obtenir 50% de fixation avec un immunsérum dont le taux de dilution est de $10^4$.

On constate ainsi que le composé de l'invention obtenu dans l'exemple 5 présente de nombreux avantages par rapport au composé obtenu dans cet exemple. En effet, le composé de l'exemple 5 est purifié rapidement, ne s'accroche pas sur les tubes et pipettes en plastique, et il permet d'obtenir un gain important en sensibilité et une économie d'anticorps par un facteur de 100.

Bien entendu, les exemples donnés ci-dessus n'ont aucun caractère limitatif vis-à-vis de l'invention. Et l'on pourraît préparer de la même façon des composés iodés conformes à l'invention en utilisant des dipeptides autres que le glycyl-tyrosine, par exemple, des composés de la tyrosine avec des peptides tels que la leucine, l'isoleucine, l'alanine, la sésrine, la tyrosine, la phénylalanine, la thréonine, la valine, l'histidine, la proline, la lysine, l'arginine, l'acide aspartique, l'acide glutamique, l'aspartamine, la glutamine.

Les composés iodés conformes à l'invention présentent cet avantage que la présence du dipeptide n'altère pas fondamentalement le caractère de l'antigène et permet d'utiliser lors des

séparations ultérieures au cours de la méthode de radio-immunologie tout les procédés de séparation applicables aux petites molécules (adsorption sur charbon, dialyse, etc...) De plus, le fait que, dans les composés de l'invention, le groupement tyrosine soit placé en position C terminale écarte au maximum, au cours de la méthode radio-immunologique, l'anticorps de la partie tyrosine iodée, dont l'encombrement pourraît gêner la liaison antigène marqué-anticorps. Les composés de l'invention sont, de plus, très solubles dans l'eau.

**Revendications**

1. Composé iodé utilisable comme traceur en radioimmunologie en tant qu'antigène marqué, caractérisé en ce qu'il est constitué par un composé conjugué d'un dipeptide et d'un composé choisi parmi un antigène présentant une fonction carboxylique libre et l'acide 5-hydroxy-indole-acétique, ledit dipeptide étant constitué par un produit de condensation entre la tyrosine et un peptide, le groupement tyrosine dudit dipeptide étant marqué à l'iode radioactif.

2. Composé selon la revendication 1, caractérisé en ce que le dipeptide est constitué par un produit de condensation de la tyrosine avec un peptide choisi dans le groupe comprenant la glycine, la leucine, l'isoleucine, l'alanine, la sérine, la tyrosine, la phényl-alanine, la thréonine, la valine, l'histidine, la proline, la lysine, l'arginine, l'acide glutamique, l'acide aspartique, l'aspartamine, la glutamine.

3. Composé selon la revendication 2, caractérisé en ce que le dipeptide est constitué par le glycyl-tyrosine.

4. Composé selon l'une quelconque des revendications 2 ou 3, caractérisé en ce que l'antigène est constitué par l'hormone juvénile.

5. Composé selon l'une quelconque des revendications 2 ou 3, caractérisé en ce que l'antigène est constitué par une prostaglandine.

6. Composé selon l'une quelconque des revendications 2 ou 3, caractérisé en ce que l'antigène est un dérivé succinylé de la sérotonine.

7. Composé selon l'une quelconque des revendications 2 ou 3, caractérisé en ce que l'antigène est l'acide abscissique.

8. Composé selon l'une quelconque des revendications 2 ou 3, caractérisé en ce que l'antigène est la progestérone.

9. Composé selon l'une quelconque des revendications 2 ou 3, caractérisé en ce que le composé iodé est un dérivé iodé analogue de la sérotonine, constitué par un composé conjugué de l'acide 5-hydroxy-indole- acétique et du dipeptide glycyl-tyrosine, le groupement tyrosine étant marqué à l'iode radioactif.

10. Procédé de préparation d'un composé selon l'une quelconque des revendications 1 à 9, caractérisé en ce qu'il comporte une réaction de condensation d'un antigène ou de l'acide 5-hydroxy-indole-acétique avec un dipeptide, ledit dipeptide étant constitué par un produit de condensation entre la tyrosine et un peptide, et une réaction d'iodation du groupement tyrosine.

11. Procédé selon la revendication 10, caractérisé en ce que l'antigène ou l'acide 5-hydroxy-indole-acétique est préalablement activé par du chloroformate d'éthyle.

12. Procédé selon l'une quelconque des revendications 10 ou 11, caractérisé en ce que l'on effectue, dans un premier temps, la réaction de condensation entre l'antigène et le dipeptide, et, dans un deuxième temps, la réaction d'iodation du groupement tyrosine.

13. Procédé selon l'une quelconque des revendications 10 ou 11, caracté-risé en ce que l'on effectue dans un premier temps l'iodation du groupement tyrosine du dipeptide et dans un deuxième temps la condensation du dipeptide ainsi marqué avec l'antigène ou l'acide 5-hydroxy-indole-acétique.

**Patentansprüche**

1. Jodhaltige Verbindung, die in der Radioimmunologie als Spurstoff verwendbar ist, als markiertes Antigen, dadurch gekennzeichnet, daß sie besteht aus einer Konjugat-Verbindung aus einem Dipeptid und einer Verbindung, die ausgewählt wird aus einem Antigen mit einer freien Carboxylfunktion und 5-Hydroxy-indol-essigsäure, wobei das Dipeptid das Kondensationsprodukt zwischen Tyrosin und einem Peptid darstellt, in dem die Tyrosingruppe des Dipeptids mit radioaktivem Jod markiert ist.

2. Verbindung nach Anspruch 1, dadurch gekennzeichnet, daß es sich bei dem Depiptid um ein Kondensationsprodukt von Tyrosin und einem Peptid handelt, das ausgewählt wird aus der Gruppe Glycin, Leucin, Isoleucin, Alanin, Serin, Tyrosin, Phenylalanin, Threonin, Valin, Histidin, Prolin, Lysin, Arginin, Glutaminsäure, Asparaginsäure, Asparaginamin und Glutamin.

3. Verbindung nach Anspruch 2, dadurch gekennzeichnet, daß es sich bei dem Dipeptid um Glycyltyrosin handelt.

4. Verbindung nach Anspruch 2 oder 3, dadurch gekennzeichnet, daß es sich bei dem Antigen um das Juvenil-Hormon handelt.

5. Verbindung nach Anspruch 2 oder 3, dadurch gekennzeichnet, daß es sich bei dem Antigen um

ein Prostaglandin handelt.

6. Verbindung nach Anspruch 2 oder 3, dadurch gekennzeichnet, daß es sich bei dem Antigen um ein Succinylderivat von Serotonin handelt.

7. Verbindung nach Anspruch 2 oder 3, dadurch gekennzeichnet, daß es sich bei dem Antigen um Abscissinsäure handelt.

8. Verbindung nach Anspruch 2 oder 3, dadurch gekennzeichnet, daß es sich bei dem Antigen um Progesteron handelt.

9. Verbindung nach Anspruch 2 oder 3, dadurch gekennzeichnet, daß es sich bei der jodhaltigen Verbindung um ein zu Serotonin analoges jodhaltiges Derivat handelt, das aus einer Konjugat-Verbindung aus 5-Hydroxy-indol-essigsäure und dem Dipeptid Glycyltyrosin besteht, worin die Tyrosingruppe mit radioaktivem Jod markiert ist.

10. Verfahren zur Herstellung einer Verbindung nach einem der Ansprüche 1 bis 9, dadurch gekennzeichnet, daß es eine Kondensationsreaktion eines Antigens oder der 5-Hydroxy-indol-essigsäure mit einem Dipeptid, wobei es sich bei dem Dipeptid um ein Kondensationsprodukt von Tyrosin mit einem Peptid handelt, und eine Reaktion zur Jodierung der Tyrosingruppe umfasst.

11. Verfahren nach Anspruch 10, dadurch gekennzeichnet, daß das Antigen oder die 5-Hydroxy-indol-essigsäure vorher mit Äthylchlorformiat aktiviert worden ist.

12. Verfahren nach Anspruch 10 oder 11, dadurch gekennzeichnet, daß in einem ersten Abschnitt die Kondensationsreaktion zwischen dem Antigen und dem Dipeptid durchgeführt wird, und daß in einem zweiten Abschnitt die Reaktion zur Jodierung der Tyrosingruppe durchgeführt wird.

13. Verfahren nach Anspruch 10 oder 11, dadurch gekennzeichnet, dab in einem ersten Abschnitt die Jodierung der Tyrosingruppe des Dipeptids durchgeführt wird, und daß in einem zweiten Abschnitt die Kondensation des auf diese Weise markierten Dipeptids mit dem Antigen oder der 5-Hydroxy-indol-essigsäure durchgeführt wird.

**Claims**

1. An iodinated compound usable as radioimmunological tracer as a labeled antigen, characterized in that said compound is constituted by a conjugate compound of a dipeptide and of a compound selected among an antigen having a free carboxylic function and 5-Hydroxy-indole acetic acid, said dipeptide being constituted by a product of condensation between tyrosine and a peptide, the tyrosine group of said dipeptide being labeled with radioactive iodine.

2. A compound according to claim 1, characterized in that the dipeptide is constituted by a product of condensation of tyrosine with a peptide selected from the group comprising glycine, leucine, isoleucine, alanine, serine, tyrosine, phenylalanine, threonine, valine, histidine, proline, lysine, arginine, glutamic acid, aspartic acid, aspartamine, glutamine.

3. A compound according to claim 2, characterized in that the dipeptide is constituted by glycyl-tyrosine.

4. A compound according to any one of claims 2 or 3, characterized in that the antigen is constituted by the juvenile hormone.

5. A compound according to any one of claims 2 or 3, characterized in that the antigen is constituted by a prostaglandin.

6. A compound according to any one of claims 2 or 3, characterized in that the antigen is a succinyl derivative of serotonin.

7. A compound according to any one of claims 2 or 3, characterized in that the antigen is abscissic acid.

8. A compound according to any one of claims 2 and 3, characterized in that the antigen is progesterone.

9. A compound according to any one of claims 2 or 3, characterized in that the iodine compound is an analog iodinated derivative of serotonin constituted by a conjugate compound of 5-hydroxy-indole acetic acid and of the glycyl-tyrosine dipeptide, the tyrosine group being labeled with radioactive iodine.

10. A method of preparation of a compound according to any one of claims 1 to 9, characterized in that said method comprises a reaction involving condensation of an antigen or 5-hydroxy-indole acetic acid with a dipeptide, said dipeptide being constituted by a product of condensation between tyrosine and a peptide, and a reaction involving iodination of the tyrosine group.

11. A method according to claim 10, characterized in that the antigen or the 5-hydroxy-indole acetic acid is previously activated by ethyl chloroformate.

12. A method according to any one of claims 10 or 11, characterized in that a first step consists in carrying out the reaction involving condensation between the antigen and the dipeptide, and a second step consists in carrying out the reaction involving iodination of the tyrosine group.

13. A method according to any one of claims 10 and 11, characterized in that a first step consists in iodination of the tyrosine group of the dipeptide, and a second step consists in condensation of the dipeptide which has thus been labeled with the antigen or the 5-hydroxy-indole acetic acid.

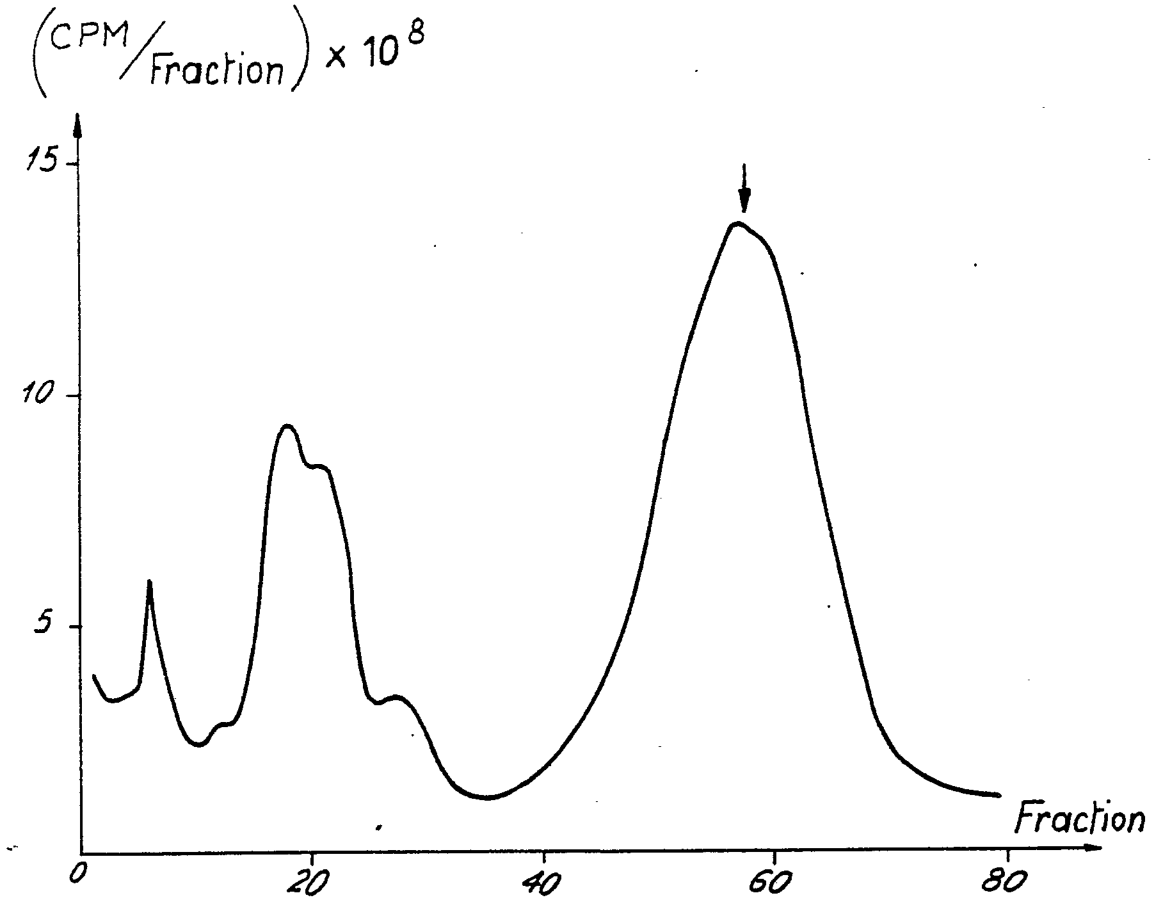
(CPM/Fraction) x 10^8
15
10
5
Fraction
0
20
40
60
80

FIG.1

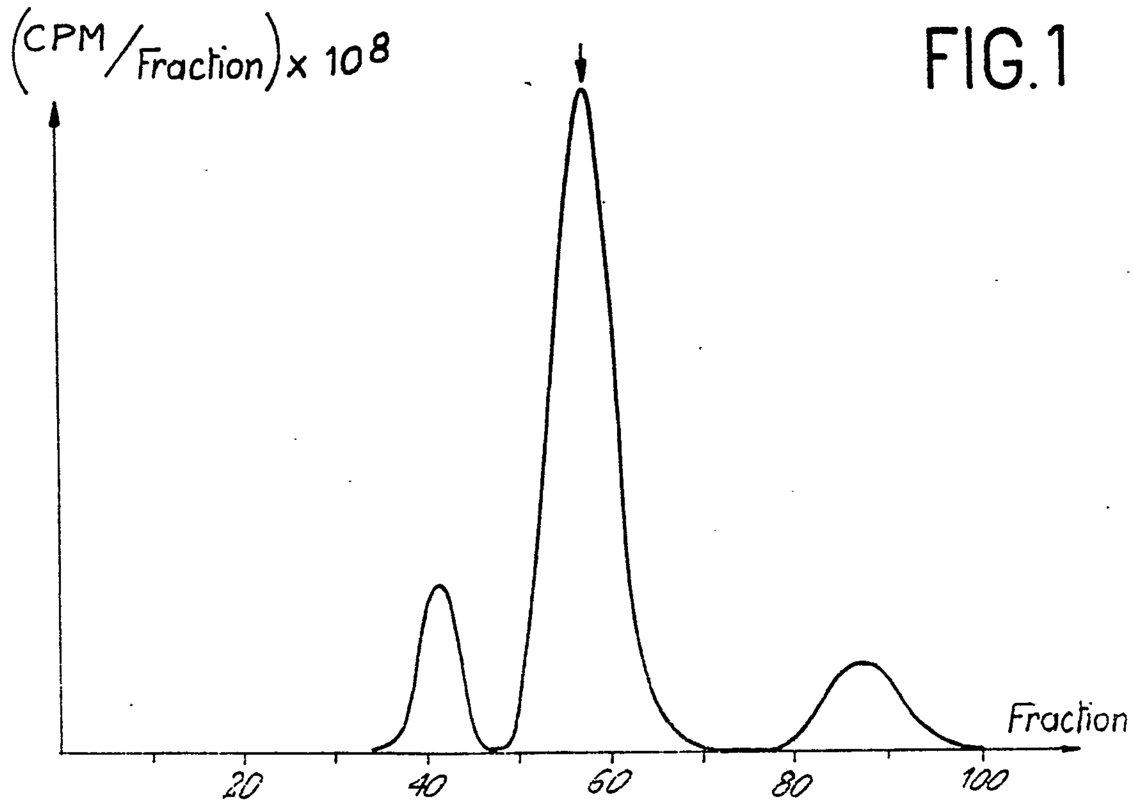
(CPM/Fraction) x 10^8
Fraction
20
40
60
80
100

FIG.3

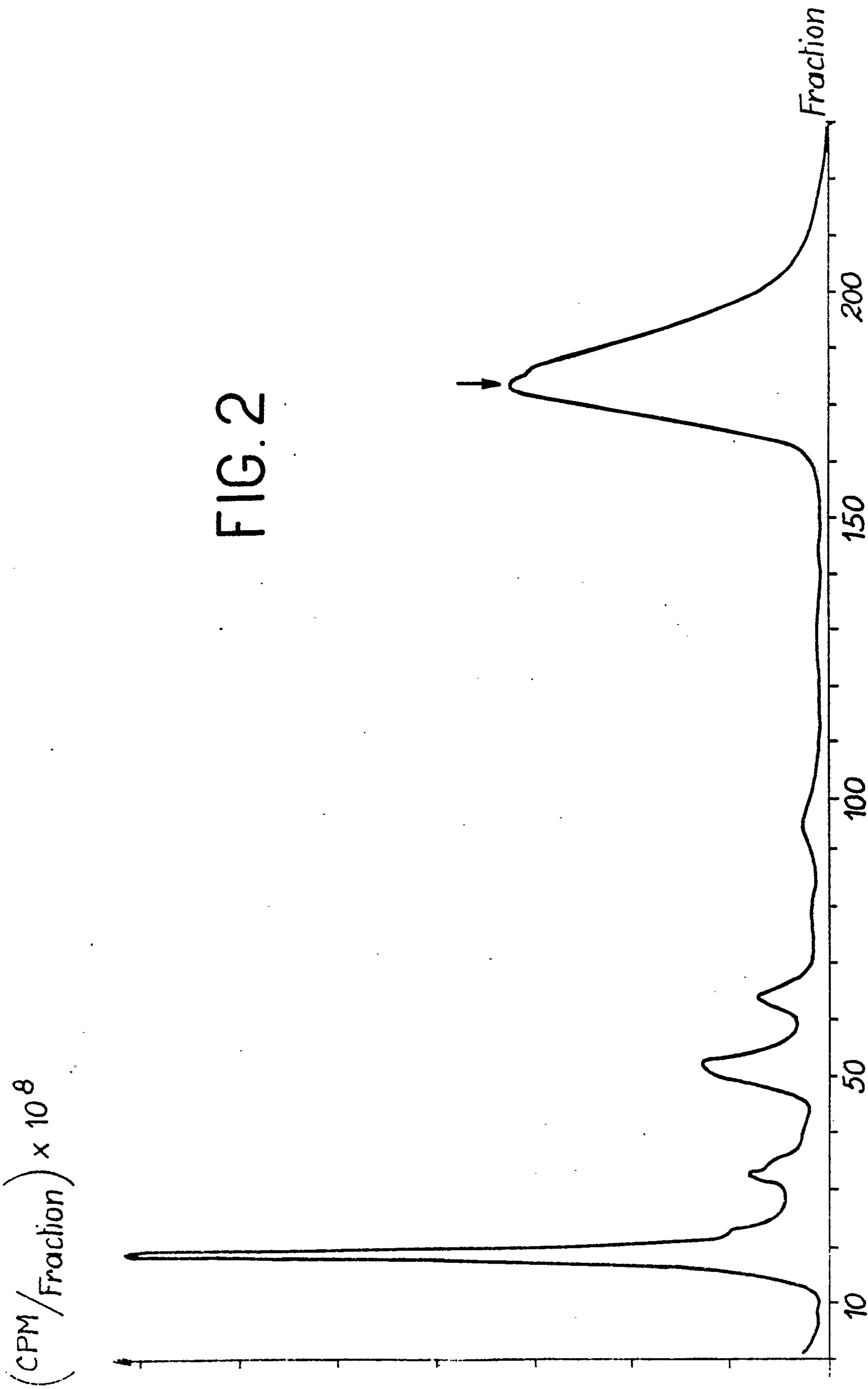


FIG. 2

FIG. 4